Europäisches Patentamt

(19) European Patent Office

Office européen des brevets

(11) Numéro de publication: **0 169 794**
**A1**

(12) # DEMANDE DE BREVET EUROPEEN

(21) Numéro de dépôt: 85430019.1

(22) Date de dépôt: 26.06.85

(51) Int. Cl.⁴: **G 01 N 33/49**

(30) Priorité: 27.06.84 FR 8410427

(43) Date de publication de la demande:
29.01.86 Bulletin 86/5

(84) Etats contractants désignés:
AT BE CH DE FR GB IT LI LU NL SE

(71) Demandeur: Girolami, Antoine
La Plaine Route de Lascours
F-13400 Aubagne(FR)

(72) Inventeur: Girolami, Antoine
La Plaine Route de Lascours
F-13400 Aubagne(FR)

(74) Mandataire: Marek, Pierre
28 & 32 rue de la Loge
F-13002 Marseille(FR)

(54) **Coagulomètre et procédé de mesure du temps de coagulation d'échantillons de produits fluides.**

(57) Procédé de mesure du temps de coagulation d'échantillons de produits fluides tels que, par exemple, d'échantillons de plasma sanguin, caractérisé en ce que l'échantillon du milieu ou produit liquide à analyser est introduit dans une petite cuve cylindrique ou tronconique (1) dont le fond (1a) est conformé pour servir de chemin de roulement circulaire (1b) à une bille (2) logée dans la partie inférieure de ladite cuve qui est animée d'un mouvement circulaire non rotatif, au moyen d'un excentrique (3-3a), entraînant une rotation de la bille suivant une trajectoire circulaire dans le fond de la cuve, le ralentissement graduel et/ou l'arrêt du mouvement rotatoire de ladite bille provoqué(s) par l'épaississement ou coagulation de l'échantillon du milieu ou produit liquide contenu dans ladite cuve, par exemple consécutif à l'introduction d'un réactif ou autre produit chimique, pouvant être constaté(s) de visu, ou de préférence, par tout système de détection automatique convenable. L'invention concerne également le coagulomètre permettant la mise en oeuvre de ce procédé.

Fig.1

Coagulomètre et procédé de mesure du temps de coagulation d'échantillons de produits fluides.

La présente invention concerne un appareil de mesure du
temps de coagulation d'échantillons de produits fluides
et plus particulièrement un coagulomètre pour la détermination du temps de coagulation d'échantillons de
plasmas sanguins ou autres tests de coagulation.
Elle vise également le procédé mis en oeuvre pour réaliser cette mesure du temps de coagulation.
Plus précisément, l'invention se rattache à un coagulomètre du genre comprenant au moins une cuve ou, le
plus souvent, une pluralité de cuves dans le fond desquelles est logée une bille animée d'un mouvement de
rotation dans un chemin de roulement circulaire ménagé
dans le fond desdites cuves.
Dans les appareils connus de ce genre (FR-A-2.318.421),
chaque cuve cylindrique est placée dans une position
inclinée et peut être entraînée en rotation autour de
son axe incliné, de sorte qu'après introduction d'un
échantillon de plasma sanguin dans ladite cuve, la
bille tourne sur place et reste, par gravité, en position déclive, tant que ledit échantillon demeure à
l'état liquide, tandis qu'elle se trouve entraînée dans
un mouvement circulaire autour de l'axe de la cuve lors
de la formation du caillot, ce changement de la position de la bille dans l'espace pouvant être constaté de
visu ou par tout système de détection automatique
approprié.

Les appareils connus de ce genre permettent d'effectuer
des mesures précises, reproductibles et simultanées.
Toutefois, après quelques années d'utilisation, il a
été constaté qu'ils étaient affectés d'un certain
nombre d'insuffisances auxquelles il était souhaitable
de remédier.

L'un de ces insuffisances découle du fait qu'il est difficile de réaliser des appareils permettant d'effectuer de nombreuses déterminations simultanées, compte tenu de la nécessité d'entraîner individuellement en rotation chaque cuve, et de la disposition inclinée de l'ensemble des cuves.

Une autre insuffisance des appareils de ce type résulte du fait qu'en raison de l'inclinaison de chaque cuve, il est difficile de déterminer le poids idéal de la bille à retenir en fonction des caractéristiques physico-chimiques des échantillons du milieu analysé.

Une autre insuffisance des appareils de ce genre, réside dans le fait qu'il est quasi impossible d'interrompre la rotation de la cuve et de la reprendre au cours d'une même détermination (par exemple pour la détermination du temps de Howell).

Une autre lacune importante de ces appareils provient du fait qu'ils ne donnent aucune information intermédiaire sur le processus de formation du caillot, entre le déclenchement et l'arrêt de la mesure qui résulte en effet de la seule détection du brusque changement de la position de la bille dans l'espace lorsque le caillot est formé.

Il est en outre difficile d'équiper ce genre d'appareil de systèmes d'introduction automatique des milieux analysés et des réactifs, notamment lorsqu'il s'agit d'appareils destinés à effectuer simultanément une pluralité de tests.

La présente invention a pour objet un procédé et un appareil remédiant aux insuffisances susmentionnées des matériels fonctionnant par détection de la position d'une bille logée dans le fond d'une cuve et permettant de s'affranchir d'un certain nombre de contraintes imposées par ce genre de matériels.

Selon l'invention, ce but est atteint grâce à un procédé suivant lequel l'échantillon du milieu ou produit liquide à analyser es: introduit dans une petite cuve cylindrique ou tronconique dont le fond est conformé pour servir de chemin de roulement circulaire à une bille logée dans la partie inférieure de ladite cuve qui est animée d'un mouvement circulaire non rotatif, au moyen d'un excentrique, entraînant une rotation de la bille suivant une trajectoire circulaire, dans le fond de la cuve, le ralentissement graduel et/ou l'arrêt du mouvement rotatoire de ladite bille provoqué(s) par l'épaississement ou coagulation dudit milieu, par exemple consécutif à l'introduction d'un réactif ou autre produit chimique, pouvant être constaté de visu ou, de préférence, par tout système de détection automatique convenable.

Le coagulomètre selon l'invention est principalement remarquable par le fait qu'il comprend : - au moins une cuve ou, de préférence, un ensemble constitué de plusieurs cuves dont le fond est conformé pour servir de chemin de roulement circulaire à une bille logée dans ladite cuve ou dans lesdites cuves ; - un dispositif à excentrique permettant de communiquer un mouvement circulaire non rotatif à la cuve ou audit ensemble de cuves ; - et des moyens assurant le guidage de ladite cuve ou dudit ensemble de cuves lors de son mouvement circulaire non rotatif.

Le procédé et l'appareil selon l'invention procurent de nombreux avantages importants.
Ce procédé offre la possibilité de mettre simultanément en mouvement, au moyen d'un seul dispositif à excentrique, un grand nombre de cuves installées sur un unique support. Ces cuves peuvent être positionnées verticalement, de sorte que le chemin de roulement circulaire

de la bille soit disposé dans un plan horizontal. Les systèmes de détection du mouvement de la bille logée dans chaque cuve peuvent être ainsi placés au-dessous des cuves. Il est de la sorte possible de réaliser des appareils permettant d'effectuer, simultanément, un grand nombre de tests ou analyses.

D'autre part, le procédé et l'appareil selon l'invention permettent d'établir des diagrammes de la décélération du mouvement circulaire de la bille en fonction de l'épaississement progressif du milieu analysé, ce qui permet, de réaliser des analyses d'une très grande précision sur le processus de formation du caillot tout au long de la durée des tests.

Les renseignements intermédiaires qu'il est ainsi possible d'obtenir peuvent être très intéressants et importants, notamment dans l'application du procédé et de l'appareil à l'analyse du temps de coagulation ou de prothrombine ou autres tests de coagulation du sang, lorsqu'on sait que ces renseignements serviront de base pour la prescription de traitements médicaux.

Un autre avantage du procédé et du coagulomètre selon l'invention réside dans le fait que le poids de la bille n'a guère d'importance et ne constitue par un facteur de perturbation de la précision des déterminations. Les cuves peuvent être positionnées à l'avance sur une plaquette-support dotée de perforations leur servant de logement ou moulées en même temps que ladite plaquette-support, de manière à constituer des ensembles standardisés composés d'un nombre déterminé de cuves. Les billes peuvent être placées à l'avance dans les cuves qui, après stérilisation, peuvent être operculées au moyen d'une bande détachable. Il est ainsi possible de n'utiliser qu'une partie des cuves constituant une plaquette de cuves.

D'autre part, la présentation des cuves sous forme de plaquettes ou tablettes facilite leur thermostatation à la température désirée avant et/ou pendant leur utilisation.

Les buts, caractéristiques et avantages susmentionnés, et d'autres encore, ressortiront mieux de la description qui suit et des dessins annexés dans lesquels :

La figure 1 est une vue de face et en coupe d'un mode d'exécution très simplifié du coagulomètre selon l'invention ; le tracé en traits interrompus illustrant la position des organes mobiles de l'appareil après un mouvement circulaire non rotatif de 180 degrés.

La figure 2 est une vue en plan de la figure 1, les trois tracés en traits interrompus illustrant les positions successives des organes mobiles de l'appareil après des mouvements circulaires non rotatifs de 90 degrés, 180 degrés et 270 degrés, respectivement.

La figure 3 est une vue en perspective éclatée d'un coagulomètre destiné à effectuer une pluralité d'analyses simultanées et équipé d'un système de détection optique.

La figure 4 est une vue partielle, de face et en coupe, d'une variante d'exécution de cet appareil.

La figure 5 est une vue partielle, de face et en coupe, d'une autre variante d'exécution du coagulomètre dont les capteurs du système de détection sont disposés latéralement par rapport aux cuves.

La figure 6 est une vue de dessous partielle, montrant un exemple de la répartition des plages alternativement transparentes et opaques de l'écran interposé entre le fond des cuves et les capteurs de l'appareil, selon un exemple d'exécution du système de détection optique équipant ce dernier.

La figure 7 est une vue partielle, en plan, montrant un exemple d'agencement du fond des cuves dotés de plages alternativement transpartentes et opaques, selon une variante de réalisation du système de détection optique

équipant le coagulomètre.

On se réfère auxdits dessins pour décrire des exemples avantageux, bien que nullement limitatifs, de mises en oeuvre du procédé et de réalisatins de l'appareil selon l'invention.

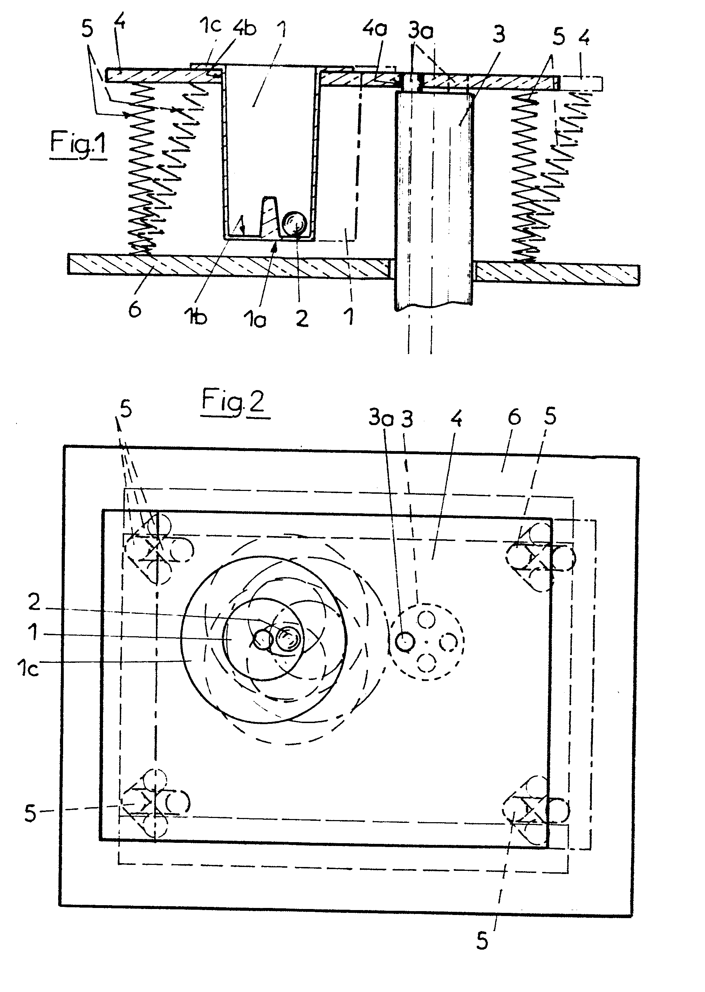

On se reporte en premier lieu aux figures 1 et 2 qui illustrent un mode d'exécution simplifié à l'extrême de cet appareil, ne comportant dans ce cas qu'une seule cuve, et qui a principalement pour but de faciliter la compréhension de la mise en oeuvre du procédé et du fonctionnement du coagulomètre selon l'invention, étant entendu que, dans la pratique et de manière avantageuse, l'appareil comporte un assez grand nombre de cuves permettant d'effectuer de nombreuses analyses simultanées, comme cela ressort de la suite du présent exposé.

Suivant le procédé de l'invention, l'échantillon du milieu ou produit liquide à analyser tel que, par exemple, un échantillon de plasma sanguin, est introduit dans une petite cuve cylindrique ou tronconique 1 dont le fond 1a est conformé pour servir de chemin de roulement circulaire 1b à une bille 2 logée dans la partie inférieure de ladite cuve qui est animée d'un mouvement circulaire non rotatif entraînant une rotation de la bille autour de l'axe de la cuve, selon une trajectoire circulaire, les différentes phases du ralentissement et/ou l'arrêt du mouvement circulaire de ladite bille provoqués par l'épaississement ou coagulation dudit milieu ou produit, par exemple consécutif à l'introduction d'un réactif ou autre produit chimique, pouvant être constatés de visu ou, de préférence, par tout système de détection automatique convenable.

L'appareil de mesure du temps de coagulation ou coagulomètre selon l'invention comprend, dans son mode
d'exécution le plus simplifié : au moins une cuve 1
dont le fond 1a est conformé pour servir de chemin de
roulement circulaire 1b à une bille 2 logée dans la
partie inférieure de ladite cuve ; - un dispositif à
excentrique permettant de communiquer un mouvement
circulaire non rotatif à la cuve ; - et des moyens
assurant le guidage de ladite cuve lors de son mouvement circulaire non rotatif.

Le dispositif à excentrique permettant de communiquer
le mouvement circulaire non rotatif à la cuve est, par
exemple, constitué par un arbre d'entraînement vertical
3 dont le sommet est équipé d'un tourillon excentré 3a
monté tournant dans un palier 4a à axe vertical dont
est pourvue une plaque ou semelle mobile 4 portant la
cuve 1 et au-dessous de laquelle est disposé ledit
arbre d'entraînement. La plaque ou semelle mobile 4 est
disposée horizontalement et elle repose, au voisinage
de ses angles, sur quatre supports flexibles verticaux,
tels que quatre ressorts hélicoïdaux 5 fixés, par leur
base, sur une table horizontale fixe 6, ces supports
flexibles tendant à reprendre leur position verticale
après flexion. La cuve 1 est disposée verticalement, de
façon que le chemin de roulement circulaire 1b ménagé
dans son fond soit placé dans un plan horizontal ou
proche de l'horizontale, cette cuve pouvant, par
exemple, reposer ou être fixée, par sa collerette
supérieure 1c, sur le bord d'un orifice circulaire 4b
réservé dans une plaquette-support qui est constituée
par la semelle 4 dans l'exemple d'exécution simplifié à
l'extrême illustré aux figures 1 et 2.

De la sorte, on conçoit que la mise en rotation de
l'arbre 3 permet de communiquer un mouvement circulaire
à la semelle mobile 4 et à la cuve 1 portée par cette
dernière, mais il s'agit d'un mouvement non rotatif, car les ressorts 5 s'opposent au mouvement révolutif de
ladite semelle tout en la guidant lors de son mouvement
circulaire.

Le mouvement circulaire non rotatif de la cuve 1 a pour
effet d'entraîner une rotation de la bille 2 dans le
fond de ladite cuve suivant une trajectoire circulaire,
ladite bille roulant, lors de ce mouvement, sur le
chemin de roulement circulaire 1b.

Lorsque l'échantillon liquide du milieu ou produit à
analyser (plasma sanguin, par exemple, dans le cas
d'analyses du temps de coagulation du sang ou de
prothrombine) est introduit dans la cuve 1 munie de la
bille 2, cette dernière se trouve noyée dans le produit
liquide et poursuit sa course rotatoire dans le fond de
ladite cuve.

L'épaississement graduel du milieu ou produit liquide,
par exemple consécutif à l'introduction d'un réactif
(thromboplastine ou autre dans le cas de mesure du
temps de coagulation du sang ou plasma sanguin) provoque, d'abord un ralentissement progressif de la
vitesse de la course rotatoire de la bille, puis
l'arrêt complet de cette dernière, cette décélération
ou cet arrêt pouvant être constatés de visu ou, de
façon beaucoup plus intéressante, au moyen de tout
système de détection approprié, disposé au-dessous de
la cuve ou latéralement par rapport à cette dernière.

Un avantage très important offert par le procédé de
l'invention est de permettre la réalisation de coagulomètres grâce auxquels il est possible d'effectuer,
de façon entièrement automatisée, de nombreuses analyses simultanées.

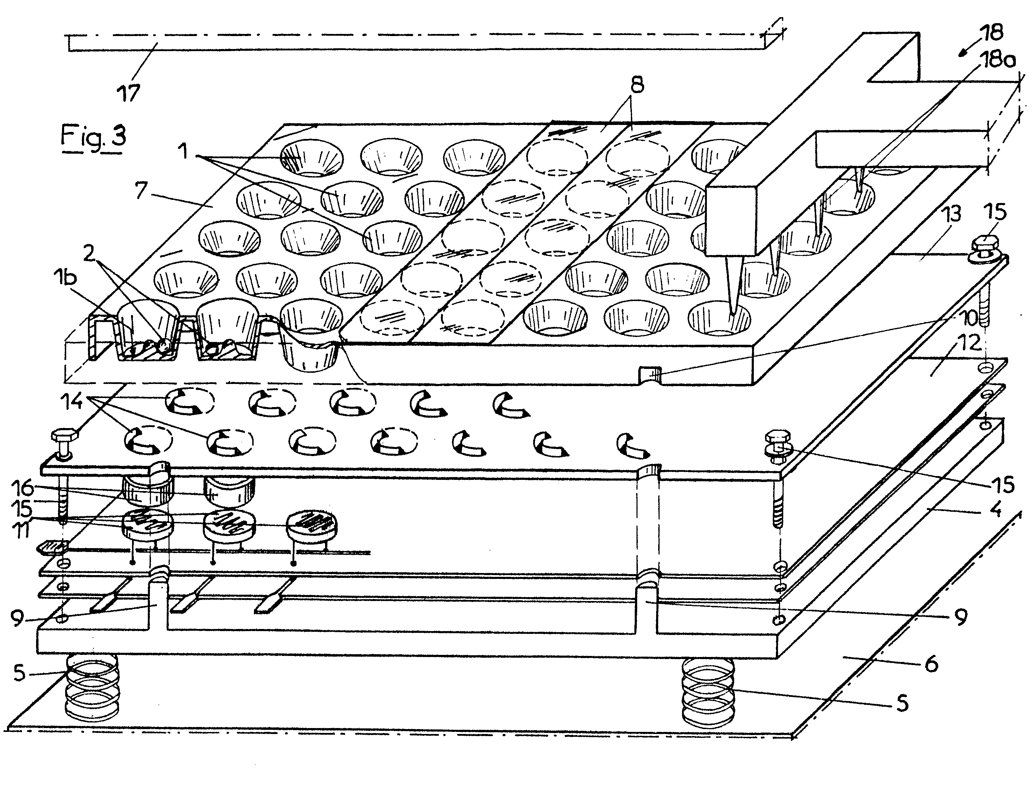

Un exemple de réalisation nullement limitatif d'un
coagulomètre de ce genre est décrit dans la suite du
présent exposé, en se référant aux figures 3 à 6. Ce coagulomètre comprend une table horizontale fixe 6 de forme rectangulaire ou autre. Sur cette table fixe, est montée une semelle mobile 4 qui est assujettie à cette dernière au moyen de quatre supports flexibles verticaux tels que, par exemple, quatre ressorts hélicoïdaux 5 reliant ladite table et ladite semelle, ces supports flexibles étant, de préférence, disposés aux angles ou au voisinage des angles de ladite semelle mobile.

Un excentrique (non représenté), par exemple du genre de celui qui est représenté aux figures 1 et 2, permet de communiquer un mouvement circulaire non rotatif à la semelle mobile 4 et aux différents organes installés sur cette dernière, l'entraînement en rotation de cet excentrique étant assuré par l'intermédiaire d'un moteur électrique et d'un réducteur, ou par tout autre moyen adéquat (non représenté).

Sur la semelle mobile, sont montés la plaquette de cuves 7 et les organes de détection du mouvement des billes par exemple disposés au-dessous de celle-ci et portés par une ou plusieurs plaques rigides ayant des formes et dimensions identiques à celles de ladite semelle mobile.

La plaquette de cuves peut être constituée par une plaque de matière plastique comportant une pluralité d'orifices circulaires servant de logement aux cuves cylindriques ou tronconiques amovibles munies d'une collerette supérieure par l'intermédiaire de laquelle elles reposent ou sont fixées sur le bord desdits orifices.

Toutefois, de manière particulièrement intéressante, la plaque et les cuves peuvent être moulées d'une seule pièce, par exemple dans une matière plastique ou autre matériau transparent ou perméable au rayon lumineux ou autre rayonnement, de façon à constituer une plaquette de cuves monobloc, composée d'un nombre relativement important de cuves 1 (40 cuves suivant l'exemple illus-

tré à la figure 3) disposées en plusieurs alignements parallèles.

Il est ainsi possible de standardiser les plaquettes de cuves.

D'autre part, les plaquettes de cuves peuvent être préparées à l'avance et livrées prêtes à l'emploi aux laboratoires utilisateurs. Dans ce cas, les billes sont placées à l'avance dans les alvéoles ou cuves 1 dont les ouvertures supérieures sont operculées au moyen de bandes détachables parallèles 8, après stérilisation des plaquettes de cuves munies de leurs billes. On conçoit que, de la sorte, il est possible, en fonction des besoins, de n'ouvrir qu'une partie des cuves d'une même plaquette de cuves, en ne détachant que les bandes de fermeture 8 de ces cuves. Ainsi, selon la figure 3, les deux alignements centraux de cuves sont représentés munis de leur opercule détachable 8.

Les billes 2 sont exécutées dans un matériau approprié à la nature du système de détection équipant l'appareil. Par exemple, lorsque le système de détection du coagulomètre comporte des capteurs utilisant l'effet opto-électronique, comme c'est le cas pour l'appareil illustré à la figure 3, les billes 2 sont exécutées dans un matériau opaque ou imperméable au rayonnement (lumineux ou autre) émis par la source de rayonnement utilisée pour agir sur les capteurs, tandis que les cuves (1) ou, tout au moins, une portion du fond 1a ou de la paroi latérale de ces dernières est réalisée dans un matériau transparent ou perméable au faisceau lumineux ou autre rayonnement fourni par la source émettrice.

La plaquette de cuves 7 et la semelle mobile 4 sont pourvues de moyens complémentaires d'assemblage, permettant l'installation amovible de ladite plaquette de cuves sur ladite semelle mobile. Ces moyens sont, par exemple, constitués, d'une part, par des colon-

exemple constitués par des diodes réceptrices ou par des cellules photoconductrices disposées de manière qu'au-dessous du fond de chaque cuve 1 de la plaquette de cuves 7, se trouve une diode réceptrice. Les capteurs 11 tels que diodes réceptrices sont avantageusement portés par une plaque rigide 12, de matière isolante à laquelle sont incorporés les circuits et les moyens de connexion desdits capteurs à l'appareil de lecture chronométrique ou autre appareil enregistreur.

Entre l'espace orbital nécessaire au mouvement rotatoire de chaque bille 2 et la diode réceptrice ou autre cellule photoconductrice 11 affectée à la détection des variations du mouvement de cette bille, est disposé un écran opaque au rayonnement (lumière monochromatique ou lumière blanche, par exemple) destiné à être capté par ladite diode ou cellule photoconductrice, cet écran comportant au moins une plage transparente audit rayonnement disposée au-dessous d'une portion du chemin de roulement circulaire 1b de chaque cuve 1.

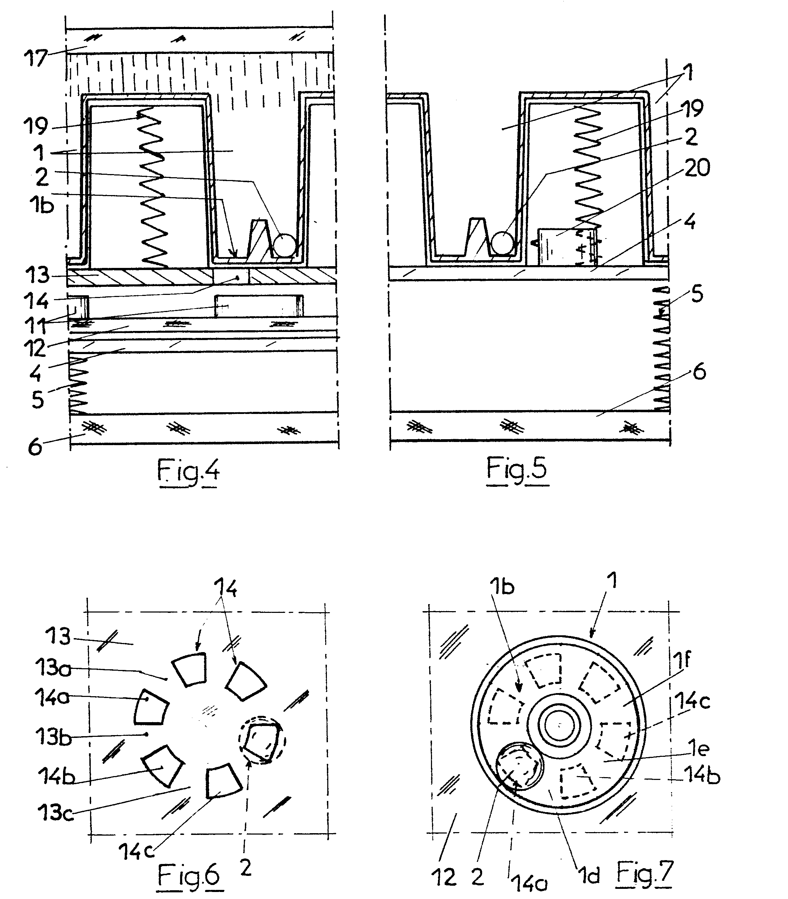

Selon le mode d'exécution illustré aux figures 3 et 4, un écran 13 est disposé entre le fond des cuves 1 de l'ensemble de cuves 7 et l'ensemble de diodes réceptrices ou cellules photoconductrices sous-jacentes 11, cet écran comportant des plages 14 transparentes audit rayonnement et disposées au-dessous des chemins de roulement circulaires 1b des billes 2. Ces plages transparentes 14 ont, de préférence, une orientation radiale par rapport aux axes alignés des cuves et des diodes réceptrices.

Il peut être prévu, entre chaque chemin de roulement 1b et chaque diode réceptrice 11, une unique plage transparente 14 ayant par exemple la forme d'une portion de couronne circulaire (figure 3) ou une pluralité de plages transparentes 14a, 14b, 14c,..., ayant la forme de secteurs ou segments de couronne circulaire et séparées par des plages opaques 13a, 13b, 13c,...,

nettes 9 dont sont pourvus au moins deux côtés opposés de la semelle mobile et, d'autre part, par des encoches latérales 10 que présente la plaquette de cuves et dans lesquelles sont engagés les sommets desdites colonnettes, lorsque ladite plaquette de cuves est installée sur ladite semelle.

Dans cette position, les chemins de roulement circulaires 1b constitués par les fonds des alvéoles ou cuves 1 de la plaquette de cuves 7, sont disposés dans un plan horizontal ou proche de l'horizontale.

Le coagulomètre comprend un système de détection automatique qui peut être de différents types (optique, à fibres optiques, magnétique, capacitif ou autre). Les capteurs de ce système de détection peuvent, suivant la nature de ce dernier, être placés au-dessous (figures 3 et 4) de l'espace orbital nécessaire au mouvement rotatoire des billes, ou latéralement par rapport à cet espace orbital (figure 5).

Le système de détection comprend une pluralité d'organes de détection individuelle reliés à un appareil de lecture chronométrique ou autre appareil enregistreur ou analyseur.

Chaque organe de détection individuelle permet de détecter les différentes variations du mouvement de la bille logée à l'intérieur de la cuve à laquelle il est associé, du début de son ralentissement jusqu'à l'arrêt complet de sa rotation.

On a illustré, à la figure 3, un exemple de réalisation d'un système de détection comportant une source de rayonnement (lumineux ou autre) et des capteurs utilisant un effet opto-électronique et permettant d'enregistrer et de transmettre les variations de potentiel électrique engendrées par le mouvement des billes disposées entre ladite source de rayonnement et lesdits capteurs.

Ce système comprend une pluralité de capteurs 11 par (figure 6), cette dernière disposition permettant des mesures extrêmement précises du ralentissement ou de la variation de la vitesse de rotation de la bille.

Les plages transparentes 14 peuvent être constituées par de simples ouvertures ménagées dans la plaque constituant l'écran 13.

La plaque 12 portant les diodes réceptrices 11 et la plaque constituant l'écran 13 sont rigidement fixées à la semelle mobile 4, par exemple au moyen de boulons dont les tiges 15 traversent des orifices alignés réservés aux angles desdites plaques. Des entretoises 16 peuvent être placées entre les plaques 12 et 13, afin de ménager un certain écartement entre ces derniè- res et d'éviter l'écrasement des diodes réceptrices 11 ou autres cellules photoconductrices.

On conçoit que lorsque la plaque de cuves 7 est installée sur la semelle, ladite plaque de cuves, l'écran 13, la plaque de diodes 11-12 et ladite semelle, sont immobilisées les unes par rapport aux autres et forment un ensemble compact qui peut être animé d'un mouvement circulaire non rotatif au moyen de l'excentrique 3-3a. L'écran doté d'une ou plusieurs plages transparentes peut également être constitué par la portion de paroi de chaque cuve disposée entre l'espace orbital néces- saire au mouvement rotatoire de la bille et le capteur du système de détection et, par exemple, comme le montre la figure 7, par la portion du fond de ladite cuve qui constitue le chemin de roulement circulaire 1b de ladite bille. Une telle disposition permet de sim- plifier la construction de l'appareil, par la suppres- sion de la plaque 13.

Selon ce mode d'exécution, la ou les plages opaques 1d, 1e, 1f, ..., et la ou les plages transparentes 14a, 14b, 14c, ..., dont est pourvu le fond 1a des cuves 1, peuvent être obtenues lors du moulage desdites cuves, ou rapportées par collage ou autrement sur ledit fond, postérieurement au moulage.

Le système de détection optique comprend encore une source de rayonnement (lumière monochromatique, lumière blanche, par exemple) désignée dans son ensemble par la référence 17 à la figure 3 et disposée au-dessus de l'ensemble mobile constitué par la semelle 4, le système de détection 11-12-13-14 et les cuves 1.

Comme indiqué précédemment, le système de détection peut aussi être constitué par une pluralité de capteurs magnétiques. Dans ce cas, les capteurs 20 peuvent être installés sur la semelle mobile 4 et disposés latéralement par rapport à l'espace orbital nécessaire au mouvement rotatoire des billes, comme le montre la figure 5, de manière à enregistrer chaque passage des billes devant lesdits capteurs.

L'appareil selon l'invention est équipé d'un dispositif permettant l'introduction automatique des milieux ou produits liquides à analyser et des réactifs, dans les cuves 1. Ce dispositif qui est désigné par la référence 18 à la figure 3, comporte une tête qui peut se déplacer au-dessus de l'ensemble de cuves 1 et qui est dotée d'une pluralité de becs 18a permettant l'introduction simultanée d'une pluralité d'échantillons de produit liquide ou de doses de réactif dans un nombre correspondant de cuves 1 disposées en alignement.

La mise en action du dispositif d'introduction du produit à analyser et/ou du réactif déclenche la mise en marche de l'instrument de chronométrie ou autres appareils enregistreurs.

On ne décrit pas en détail, l'instrument de chronométrie, la source de rayonnement et le dispositif d'introduction des milieux à analyser et/ou des réactifs qui peuvent être de différents types connus.

On conçoit bien le fonctionnement de l'apparteil selon l'invention que l'on expose ci-après en se référant au mode d'exécution illustré à la figure 3.

Lorsque l'ensemble constitué par la semelle 4, le système de détection 11-12-13-14 et la plaque de cuves 7 est animé d'un mouvement circulaire non rotatif au moyen de l'excentrique 3-3a, les billes 2 logées dans les cuves sont entraînées en rotation selon une trajectoire circulaire autour de l'axe desdites cuves, qu'il y ait ou non le produit à analyser seul ou le réactif seul dans le fond de ces dernières.

Comme indiqué, l'introduction du réactif, ou du milieu liquide à analyser, à l'aide du dispositif d'introduction automatique 18 déclenche la mise en marche de l'instrument de chronométrie ou autre appareil enregistreur recevant les signaux fournis par les capteurs.

Dans un premier temps, la bille 2 de chaque unité d'analyse passe successivement et régulièrement au-dessus des plages transparentes et opaques de l'écran 13 et intercepte périodiquement, partiellement ou complètement, le faisceau lumineux ou autre faisceau de rayons émis par la source de rayonnement et atteignant la diode réceptrice 11. Ces passsages périodiques réguliers provoquent des variations régulières de potentiel électrique au niveau de ladite diode réceptrice, lesquelles sont transmises au système de lecture de l'appareil de chronométrie ou autre appareil enregistreur et se traduisent par des courbes régulières constituées d'une alternance de sommets et de creux ; le mouvement de rotation de la bille étant uniforme, le temps qui sépare deux pics ou sommets successifs ou deux creux successifs est constant.

Le processus de coagulation ou de formation du caillot qui se constate par un épaississement graduel du plasma sanguin ou autre milieu, entraîne un ralentissement progressif du mouvement circulaire de la bille jusqu'à l'arrêt complet de cette dernière. Ce ralentissement

progressif provoque une modification des caractéristiques des variations de potentiel électrique au niveau de la diode réceptrice, lesquelles se traduisent, sur le système de lecture de l'appareil enregistreur par un allongement du temps entre deux pics ou creux successifs.

Grâce au procédé et à l'appareil selon l'invention, il est possible d'enregistrer toutes les variations de la vitesse de rotation de la bille durant le processus de coagulation, ce qui permet des analyses de ce processus nouvelles et précises.
L'arrêt du mouvement de rotation de la bille entraîne la suppression des variations de potentiel électrique au niveau de la diode réceptrice, cette situation provoque l'arrêt de l'unité de chronométrie et/ou d'enregistrement affectée à l'unité de détection concernée.

Le coagulomètre selon l'invention peut également comporter une plaque chauffante, alvéolée et thermostatée pour la réception des cuves. Cette plaque disposée au-dessous des cuves est rigidement fixée à la semelle mobile 4. Cette plaque pourrait être indépendante ou constituée par la plaque-écran 13 pourvue de résistances 19 (figure 4) et équipée d'un thermostat. Lorsque la nature du système de détection le permet, elle peut être aussi directement constituée par la semelle mobile 4 agencée et équipée à cet effet.

Revendications

1. - Procédé de mesure du temps de coagulation d'échantillons de produits fluides tels que, par exemple, d'échantillons de plasma sanguin, caractérisé en ce que l'échantillon du milieu ou produit liquide à analyser est introduit dans une petite cuve cylindrique ou tronconique (1) dont le fond (1a) est conformé pour servir de chemin de roulement circulaire (1b) à une bille (2) logée dans la partie inférieure de ladite cuve qui est animée d'un mouvement circulaire non rotatif, au moyen d'un excentrique (3-3a), entraînant une rotation de la bille suivant une trajectoire circulaire dans le fond de la cuve, le ralentissement graduel et/ou l'arrêt du mouvement rotatoire de ladite bille provoqué(s) par l'épaississement ou coagulation de l'échantillon du milieu ou produit liquide contenu dans ladite cuve, par exemple consécutif à l'introduction d'un réactif ou autre produit chimique, pouvant être constaté(s) de visu, ou de préférence, par tout système de détection automatique convenable.

2. - Procédé selon la revendication 1, caractérisé en ce que le chemin de roulement circulaire (1b) ménagé dans le fond (1a) de la cuve (1) est disposé dans un plan horizontal ou sensiblement horizontal et en ce que la détection du mouvement circulaire, de la décélération et/ou de l'arrêt du mouvement rotatif de la bille, est opérée au moyen d'un capteur (11) disposé au-dessous de ladite cuve (1) ou latéralement par rapport à cette dernière.

3. - Procédé suivant la revendication 2, selon lequel la détection du mouvement circulaire, de la décélération et/ou de l'arrêt du mouvement rotatif de la ou des billes (2) est opérée au moyen d'une installation

- 2 -                    0169794

comportant une source de rayonnement (17) et un ou plusieurs capteurs (11) utilisant l'effet photo-électrique, caractérisé en ce que l'on enregistre les variations de potention électrique engendrées par le ou les capteurs et résultant du passage périodique de la bille ou de chaque bille (2) devant une ou plusieurs plages (14) transparentes au rayonnement émis par la source de rayonnement (17) et que présente un écran opaque (13) disposé entre l'espace orbinal nécessaire au mouvement rotatoire de la bille ou de chaque bille et le ou les capteurs (11) et dans le champ du faisceau de rayons émis par ladite source de rayonnement.

4. - Coagulomètre caractérisé en ce qu'il comprend :
- au moins une cuve (1) ou, de préférence, un ensemble constitué de plusieurs cuves (1) dont le fond (1a) est conformé pour servir de chemin de roulement circulaire (1b) à une bille logée dans ladite cuve ou dans lesdites cuves ;
- un dispositif à excentrique (3-3a) permettant de communiquer un mouvement circulaire non rotatif à ladite cuve ou audit ensemble de cuves ;
- et des moyens (5, 6) assurant le guidage de ladite cuve ou dudit ensemble de cuves lors de son mouvement circulaire non rotatif.

5. - Coagulomètre selon la revendication 4 comportant un système de détection relié à un instrument de chronométrie et/ou à un appareil enregistreur, caractérisé en ce que le chemin de roulement circulaire (1b) ménagé dans le fond (1a) de la cuve ou de chaque cuve de l'ensemble de cuves, est disposé dans un plan horizontal ou proche de l'horizontale, dans sa position fonctionnelle, et en ce que le ou les capteurs (11) du système de détection est ou sont disposé(s) au-dessous de l'espace orbital nécessaire au mouvement rotatoire de la ou des billes (2) ou latéralement par rapport audit espace orbital.

6. - Coagulomètre suivant la revendication 5 dont le système de détection comprend une source de rayonnement lumineux ou autre rayonnement, caractérisé en ce que cette source de rayonnement (17) est disposée au-dessus de la cuve (1) ou de l'ensemble de cuves (1).

7. - Coagulomètre selon l'une des revendications 5 ou 6 dont le système de détection comprend un ou plusieurs capteurs ou récepteurs (11) tel(s) qu'une ou plusieurs diodes réceptrices aptes à engendrer et à signaler les variations de potentiel électrique provoquées par l'occultation partielle ou totale d'un faisceau de rayons émis par la source de rayonnement (17) caractérisé en ce qu'un écran opaque (13) est disposé entre l'espace orbital nécessaire au mouvement circulaire de la ou des billes (2) et le ou les capteurs (11) et dans le champ du faisceau de rayons émis par ladite source de rayonnement (17), ledit écran étant pourvu d'une ou plusieurs plages (14) transparente(s) au rayonnement émis par cette dernière.

8. - Coagulomètre selon la revendication 7, caractérisé en ce que l'écran opaque pourvu d'une ou plusieurs plages transparentes est constitué par la portion de paroi de chaque cuve interposée entre l'espace orbital nécessaire au mouvement rotatoire de la bille logée dans ladite cuve et le capteur affecté à la détection des variations du mouvement de ladite bille et, de préférence, par la portion du fond (1a) de ladite cuve constituant le chemin de roulement (1b) de ladite bille.

9. - Coagulomètre suivant la revendication 4, caractérisé en ce qu'il comporte une semelle mobile (4) destinée à la réception de la cuve (1) ou de la plaquette ou ensemble de cuves (7) et recevant le mouvement de l'excentrique (3-3a), ladite semelle mobile étant portée par des supports flexibles (5) fixés par leur base, à une table fixe.

10. - Coagulomètre selon la revendication 9, caractérisé en ce qu'il comporte une plaque chauffante thermostatée (13-19) disposée au-dessous de la plaquette de cuves (7).

11. - Coagulomètre selon l'une des revendications 9 ou 10, caractérisé en ce que les organes de détection (11, 13, 14) du mouvement de la bille ou des billes (2) et/ou la plaque chauffante thermostatée (13-19) sont rigidement fixés sur la semelle mobile (4).

Fig.1

Fig.2

0169794

Fig.3

3/3

0169794

Fig.4

Fig.5

Fig.6

Fig.7

**Office européen des brevets**

## RAPPORT DE RECHERCHE EUROPEENNE

### DOCUMENTS CONSIDERES COMME PERTINENTS

| Catégorie | Citation du document avec indication. en cas de besoin. des parties pertinentes | Revendication concernee | CLASSEMENT DE LA DEMANDE (Int. Cl 4) |
|---|---|---|---|
| Y | FR-A-2 465 227  (HEINRICH AMELUNG)<br>* Page 5, ligne 5 - page 8, ligne 30 * | 1,4 | G 01 N   33/49 |
| A | | 2,5 | |
| | --- | | |
| Y | FR-A-2 089 651  (FRITZ HELLIGE)<br>* Page 4, ligne 33 - page 5, ligne 28 * | 1 | |
| A | | 4,10 | |
| | --- | | |
| Y | EP-A-0 037 955  (SCIENTIFIC MANUFACTURING INDUSTRIES)<br>* Page 3, lignes 17-27 * | 4 | |
| | | | **DOMAINES TECHNIQUES RECHERCHES (Int. Cl.4)** |
| A | | 10 | G 01 N   33/00<br>G 01 N   11/00<br>B 01 L   11/00 |
| | --- | | |
| A | US-A-4 054 151  (PARKER et al.)<br><br>* Colonne 3, lignes 8-37 * | 1,4,10 ,11 | |
| | --- | | |
| A | FR-A-2 516 658  (BEHNK et al.)<br><br>* Page 5, ligne 32 - page 6, ligne 28; page 13, ligne 22 - page 14, ligne 5 * | 1,2,5, 9 | |
| | ---          -/- | | |

Le présent rapport de recherche a été établi pour toutes les revendications

| Lieu de la recherche | Date d'achèvement de la recherche | Examinateur |
|---|---|---|
| LA HAYE | 07-10-1985 | BINDON C.A. |

**0169794**

Numero de la demande

EP 85 43 0019

## DOCUMENTS CONSIDERES COMME PERTINENTS

| Catégorie | Citation du document avec indication, en cas de besoin, des parties pertinentes | Revendication concernee | CLASSEMENT DE LA DEMANDE (Int. Cl 4) |
|---|---|---|---|
| A | FR-A-2 318 421 (GIROLAMI)<br><br>* Page 5, ligne 6 - page 8, ligne 2 *<br><br>----- | 1-3,5, 7-9 | |
| | | | **DOMAINES TECHNIQUES RECHERCHES (Int. Cl.4)** |

Le présent rapport de recherche a été établi pour toutes les revendications

| Lieu de la recherche | Date d'achevement de la recherche | Examinateur |
|---|---|---|
| LA HAYE | 07-10-1985 | BINDON C.A. |